(19) Europäisches Patentamt
European Patent Office
Office européen des brevets

(11) Publication number: **0 193 386**
**A2**

(12) **EUROPEAN PATENT APPLICATION**

(21) Application number: 86301360.3

(22) Date of filing: 26.02.86

(51) Int. Cl.[4]: C 11 D 1/94

(30) Priority: 28.02.85 US 706573

(43) Date of publication of application: 03.09.86 Bulletin 86/36

(84) Designated Contracting States: BE DE FR IT LU NL SE

(71) Applicant: THE PROCTER & GAMBLE COMPANY
One Procter & Gamble Plaza
Cincinnati Ohio 45202(US)

(72) Inventor: Mao, Mark Hsiang-Kuen
4114 Fox Hollow Drive
Cincinnati Ohio 45241(US)

(72) Inventor: Bernardino, Lowell Watson
6511 Edwood Avenue
Cincinnati Ohio 45224(US)

(72) Inventor: Bissett, Donald Lynn
3925 Dust Commander Drive
Hamilton Ohio 45011(US)

(72) Inventor: Fisher, Patrice
1531 Nathanial Drive
Cincinnati Ohio 45240(US)

(74) Representative: Brooks, Maxim Courtney et al,
Procter & Gamble (NTC) Limited Whitley Road
Longbenton
Newcastle-upon-Tyne NE12 9TS(GB)

(54) **Mild detergent compositions.**

(57) Mild detergent compositions comprise mild, water soluble, foaming anionic detergent surfactants and a mixture of small amounts of betaine surfactant and chelating agent selected from the group consisting of salts of citric acid, pyrophosphoric acid, tripolyphosphoric acid, maleic acid, succinic acid, malonic acid, tartaric acid, malic acid, saccharic acid, ether polycarboxylates, and mixtures thereof.

EP 0 193 386 A2

# MILD DETERGENT COMPOSITIONS

Mark H. K. Mao
Donald L. Bissett
Lowell Watson Bernardino
and
Patrice Fisher

## Technical Field & Background Art

The invention relates to mild detergent compositions containing mild detergent surfactants and a mixture of a low level of betaine surfactant, optional amine oxide or amide suds booster and specific chelating agent effective at neutral and near neutral pHs for consumer preferred skin condition.

Mild detergent compositions are well known in the art. Typically mildness is achieved by the use of sulfates of highly ethoxylated alcohols, see e.g. U.S. Patent 3,743,233, Rose & Thiele, incorporated herein by reference. Betaines have also been suggested for use in compositions for washing dishes, see e.g., U.S. Patent 4,166,048, Nishimura et al and U.S. Patent 4,137,191. Citric acid and citrates are known for use in light duty detergents, see e.g., U.S. Patent 3,650,968, Hoffman et al, and British Patent 2,010,892, Reilly et al. All of said patents are incorporated herein by reference.

## Summary of the Invention

The present invention relates to a mild foaming detergent composition comprising:

(1) from about 5% to about 99% of mild, water soluble, foaming anionic detergent surfactant selected from the group consisting of: compounds having the formula $RE_xS$ wherein R is an alkyl group containing from about 10 to about 18 carbon atoms or an alkyl phenyl group in which the alkyl contains from about 5 to about 13 carbon atoms, E represents an ethylene oxide moiety, x is a number from about 1 to about 20 on the average,

and S is a neutralized sulfate group; $C_{10-16}$ alkylglycerylether sulfonates; and mixtures thereof;

(2) from about 1% to about 30% of a betaine surfactant having the formula

$$R-\overset{(+)}{N}(R^1)_2-R^2\overset{(-)}{COO}$$

wherein R contains from about 12 to about 20 carbon atoms, $R^1$ contains from one to about 3 carbon atoms and $R^2$ contains from one to about 6 carbon atoms;

(3) from 0% to about 20% of an amine oxide suds booster;

(4) from 0% to about 20% of a fatty acid amide suds booster; and

(5) from about 0.5% to about 50% of chelating agent effective at neutral and near netural pH's, preferably selected from the group consisting of salts of citric acid, pyrophosphoric acid, malic acid, saccharic acid, maleic acid, succinic acid, malonic acid, tartaric acid, tripolyphosphoric acid, ether polycarboxylates and mixtures thereof; and

wherein, when said composition contains harsh surfactants such as $C_{10-16}$ alkyl sulfates and $C_{6-13}$ alkyl benzene sulfonates, said harsh surfactants are complexed with e.g., said amine oxide suds booster or said betaine surfactant to make them milder.

## Detailed Description of the Invention

The detergent compositions of the present invention contain three essential components:

(1) mild, water soluble, foaming anionic detergent surfactant;

(2) low level of betaine detergent surfactant; and

(3) the chelating agent.

Optional ingredients can be added to provide various performance and aesthetic characteristics.

The Mild, Water Soluble, Foaming Anionic Detergent Surfactant

The compositions of this invention contain from about 5% to about 99%, preferably from about 10% to about 40%, most preferably from about 20% to about 35% of mild, water soluble, foaming detergent surfactant.

The preferred mild anionic detergent surfactants have the generic formula $RE_xSM$ wherein R is an alkyl group containing from about 10 to about 18 carbon atoms, preferably from about 12 to about 15 carbon atoms, or, less preferred, an alkyl phenyl group in which the alkyl group contains from about 10 to about 18 carbon atoms, preferably from about 11 to about 14 carbon atoms, E represents an ethylene oxide moiety, x is a number from about 1 to about 20 on the average, preferably from about 1 to about 12, S is a sulfate group, and M is an alkali metal, alkaline earth metal, ammonium, or substituted ammonium cation.

An example of the preferred anionic synthetic detergent is the sodium, ammonium, substituted ammonium, potassium, or magnesium alkylpolyethoxylate sulfate, or mixtures thereof, obtained by sulfating the reaction product of higher alcohols containing from about 10 to about 15 carbon atoms, with from about 1 to about 20 $[C_{10-15}AE_{(1-20)}S]$, preferably from about 1 to about 12 $[C_{10-15}AE_{(1-12)}S]$ moles of ethylene oxide per mole of alcohol. Such surfactants which have a narrow distribution of ethoxylate chain lengths are preferred especially when there is very little surfactant with no ethoxylate content.

Another suitable anionic detergent surfactant is an alkylglycerylether sulfonate in the form of its sodium, potassium, magnesium, ammonium, or substituted ammonium salt, wherein the alkyl group contains from about 10 to about 18 carbon atoms, preferably from about 12 to about 16 carbon atoms.

Mixtures of all of the above detergent surfactants can be used. These mild detergent surfactants are essential to the end result, i.e., preparation of a mild detergent composition providing a consumer preferred skin condition. When harsh detergent surfactants are used, the resulting irritation tends to mask the

improved skin condition created by the mixture of the betaine detergent surfactant and chelating agent.

The Betaine Detergent Surfactant

The betaine detergent surfactant has the general formula:

$$R\text{-}\overset{(+)}{N}(R^1)_2R^2\overset{(-)}{COO}$$

wherein R is a hydrophobic group selected from the group consisting of alkyl groups containing from about 10 to about 22 carbon atoms, preferably from about 12 to about 18 carbon atoms, alkyl aryl and aryl alkyl groups containing a similar number of carbon atoms with a benzene ring being treated as equivalent to about 2 carbon atoms, and similar structures interrupted by amido or ether linkages; each $R^1$ is an alkyl or hydroxyalkyl group containing from one to about 3 carbon atoms; and $R^2$ is an alkylene group containing from one to about 6 carbon atoms.

Examples of preferred betaines are dodecylamidopropyl dimethylbetaine; dodecyldimethylbetaine; tetradecyldimethylbetaine; cetyldimethylbetaine; cetylamidopropyldimethylbetaine, tetradecyldimethylbetaine, tetradecylamidopropyldimethylbetaine, and docosyldimethylammonium hexanoate and mixtures thereof.

Betaines containing a $C_{12-14}$ alkyl are more useful in this invention, than when used by themselves. The chelating agent improves the desquamation of these betaines dramatically.

The betaine is present at a level of from about 0.5% to about 30% by weight of the formula, preferably from about 1% to about 15%, most preferably from about 1% to about 10%. The ratio of anionic detergent surfactants to the betaine is from about 1:1 to about 30:1, preferably from about 1:1 to about 20:1, more preferably from about 5:1 to about 20:1.

The Suds Boosters

Suitable amine oxide suds boosters have the formula:

$$R^1\text{-}(OR^2)_n\text{-}N(R^3)_2\rightarrow O$$

in which $R^1$ is an alkyl group of from about 8 to about 18, preferably from about 10 to about 14, carbon atoms; $R^2$ is an alkylene or a hydroxy alkylene group containing 2 to 3,

preferably 2, carbon atoms; n ranges from 0 to about 20, preferably 0; and each $R^3$ is selected from the group consisting of methyl, ethyl and hydroxyethyl groups which can be joined, e.g., to form morpholine or pyridine rings; and mixtures thereof. The arrow in the formula is a conventional representation of a semi-polar bond. Specific examples of amine oxide detergents include dodecyldimethylamine oxide, tridecyldimethylamine oxide, tetradecyldimethylamine oxide, pentadecyldimethylamine oxide, hexadecyldimethylamine oxide, heptadecyldimethylamine oxide, octadecyldimethylamine oxide, dodecyldiethylamine oxide, tetradecyldiethylamine oxide, hexadecyldiethylamine oxide, octadecyldibutylamine oxide, dodecyldibutylamine oxide, tetradecyldibutylamine oxide, octadecyldibutylamine oxide, bis(2-hydroxyethyl) dodecylamine oxide, bis-(2-hydroxyethyl)-3-dodecoxy-1-hydroxypropylamine oxide, (2-hydroxypropyl)methyltetradecylamine oxide, dimethyl(2-hydroxydodecyl)amine oxide, 3,6,9-trioxoctadecyldimethylamine oxide, and 3-dodecoxy-2-hydroxypropyldi(2- hydroxyethyl)amine oxide.

The product contains from 0% to about 20%, preferably from about 1% to about 15%, most preferably from about 1% to about 5%, of amine oxide suds booster with the ratio of anionic surfactant to amine oxide being from about 2:1 to about 20:1, preferably from about 3:1 to about 10:1.

Suitable fatty acid amide suds boosters include those having the formula

$$R\overset{O}{\overset{\|}{C}} - N\,(H)_x\,(R^1)_{2-x}$$

wherein R is an alkyl chain containing from about 7 to about 17, preferably from about 9 to about 13, carbon atoms; x is 0, 1, or 2; and each $R^1$ is an alkyl or hydroxy alkyl group containing from 1 to 3 carbon atoms. Ethoxylated amides containing up to about ten moles of ethylene oxide per mol of amide are also useful.

Examples of preferred alkanol amides include coconut ($C_{12-14}$) mono-and diethanolamides and mono-and dipropanolamides. Equivalent synthetically derived amides are also desirable. The levels of amide and ratios of anionic surfactant to amide are the same as the anionic/amine oxide ratios and amine oxide levels.

The Chelating Agents

The chelating agents of this invention cooperate with the betaines and the other ingredients to improve the mildness of the compositions of this invention. The chelating agents should effectively chelate calcium at neutral and near neutral pH's. The chelating agents include salts of citric acid, pyrophosphoric acid, maleic acid, tartaric acid, succinic acid, malonic acid, tripolyphosphoric acid, malic acid, saccharic acid, ether polycarboxylates, and mixtures thereof. These chelating agents are the water soluble salts, including partial salts, especially of the types used in light duty detergents. Sodium; potassium; ammonium; and mono-, di-, and triethanolammonium salts are preferred. Magnesium and substituted ammonium salts are desirable also. Organic chelating agents are preferred.

Ether polycarboxylates are those having the general formula:

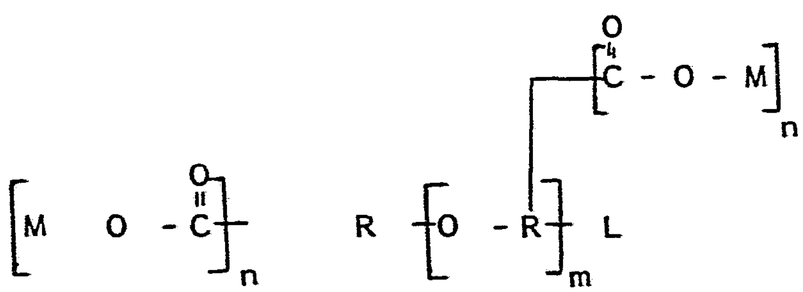

wherein each R is an alkylene group containing from one to about 4 carbon atoms or, optionally, a hydroxy alkylene group containing from one to about 3 hydroxy groups, each n is from one to about four, m is one to about 4, preferably one, M is H or a water soluble cation like Na, K, $NH_4^+$, substituted ammonium, etc; and L is either -H, -OH, or a covalent bond to an earlier R group to complete the formula.

Preferred ether polycarboxylates are

$$MO-\overset{O}{\overset{\|}{C}}-CH_2\ O-CH_2\ \overset{O}{\overset{\|}{C}}-OM$$ and

$$M-O-\overset{O}{\overset{\|}{C}}-CH_2-\underset{O=C-OM}{\underset{|}{CH}}-O-\underset{O=C-OM}{\underset{|}{CH}}-CH_2-\overset{O}{\overset{\|}{C}}-OM$$

Smaller molecules are desirable, as are hydroxy substituents, to improve solubility in product.

The level of chelating agents is from about 0.5 to about 50%, preferably from about 1 to about 20%, most preferably from about 1 to about 10%. The preferred chelating agents are salts of citric acid.

<u>The Mildness Effect</u>

It is believed that the betaine/chelating agent mixture functions primarily by providing a desquamatory action to the detergent composition. This invention is an improvement over the invention described in copending application Serial Number 623,817 of Donald L. Bissett and Mark H. K. Mao for Mild Detergent Compositions, filed June 22, 1984, now U.S. Patent 4,555,360, incorporated herein by reference. It is believed that the mixture removes damaged (e.g., dry) skin cells on the surface of the skin, thereby reducing the rough feel associated therewith. The mixture is more effective than either ingredient alone. Since these damaged skin cells would naturally sluff off eventually, the effect is merely to accelerate the natural process. The compositions herein remove the effect of prior damage to the skin, giving the skin a fresher, more youthful appearance and feel. When the betaine/chelating agent mixture is combined with a mild detergent composition, especially one that contains an amine oxide suds booster, the overall effect is to promote the health of the skin and to provide the consumer with a perceived mildness or skin feel/appearance advantage over other similar detergent compositions which do not contain the essential ingredients herein.

Optional Components

In addition to the essential ingredients described hereinbefore, the compositions can contain other conventional ingredients, especially those associated with dishwashing compositions, shampoos and handwashing compositions, e.g., "liquid soaps".

Optional ingredients include "harsh" detergent surfactants such as $C_{10-16}$ olefin sulfonates and sulfates, $C_{10-16}$ alkyl sulfates and $C_{6-13}$ alkylbenzene sulfonates, so long as they are complexed with other ingredients, e.g., the amine oxides to form mild complexes.

The compositions can also contain mild, water soluble detergent surfactants such as nonionic detergent surfactants which may not foam and may even inhibit foaming. Such nonionic detergents are disclosed in U.S. Patent 4,321,165, Smith et al (March 23, 1982), incorporated herein by reference.

Other conventional optional ingredients which are usually used in additive levels of below about 5% include opacifiers, antioxidants, bactericides, dyes, perfumes, optical brighteners and the like.

The composition can also have pH regulants present. Desirably the pH of the composition in use is from about 5 to about 10, preferably from about 6 to about 9, most preferably from about 7 to about 8. Preferably, high pHs are avoided.

Other desirable ingredients include diluents and solvents. Diluents can be inorganic salts, such as sodium sulfate, ammonium chloride, magnesium chloride, sodium chloride, sodium bicarbonate, etc., and the solvents include water, lower molecular weight alcohols such as ethyl alcohol, isopropyl alcohol, etc. In liquid detergent compositions there will typically be from 0% to about 90%, preferably from about 20% to about 70%, most preferably from about 40% to about 60% of water, and from 0% to about 50%, most preferably from about 3% to about 10% of ingredients to promote solubility, including ethyl or isopropyl alcohol, short chain polyethylene glycols, conventional hydrotropes such as ammonium toluene, xylene, or cumene sulfonates, etc.

All parts, percentages and ratios herein are by weight unless otherwise specified.

The following examples illustrate the invention.

EXAMPLE I

Farm pigs were kept in a low humidity environment for dry skin to develop. Different product solutions were then used to wash the dry skin. The removed skin flakes (scales) were collected by centrifugation and analyzed quantitatively by protein assay following a base hydrolysis step. The data shows good skin flake (scale) removal ability for formulas A and B, but the best is for B in both soft and hard water.

| | Soft Water 0 gr./gal. | | Hard Water 8 gr./gal. | |
|---|---|---|---|---|
| Composition % | A | B | A | B |
| $RE_xS$ (R = $C_{12,13}$; X = 4.3 ammonium salt) | 27 | 27 | 27 | 27 |
| Dodecyldimethylamine oxide | 5 | 5 | 5 | 5 |
| Cetyldimethylbetaine | 5 | 5 | 5 | 5 |
| Ammonium citrate | 0 | 5.6 | 0 | 5.6 |
| μg of protein/$cm^2$ skin | 607 (±216) | 827 (±154) | 501 (±133) | 702 (±92) |

EXAMPLE II

Three liquid detergents with the following compositions were tested in a hand soak test. In this test 24 panelists were used. Each panelist soaked one hand in one of the test solutions and the other hand in another different test solution for four days, 30 minutes each day. Their hand conditions were graded by two qualified skin graders before and after the soakings. Data were then analyzed statistically.

| Composition % | A | B | C |
|---|---|---|---|
| Cetyldimethylbetaine | 0.0 | 5.0 | 5.0 |
| Sodium citrate | 0.0 | 0.0 | 6.0 |
| Dodecyldimethylamine oxide | 5.0 | 5.0 | 5.0 |
| $RE_xS$ | 27.0 | 27.0 | 27.0 |
| Relative Skin Grade Change* | Control | +0.33 | +0.56** |

* Skin Grade

A is used as the control. Skin grades are based upon a standard dermatological scale in which 10 is perfect skin, normal skin ranges between 5 and 10, and the difference from one grade to the next grade is a large, readily detectable difference.

** Significantly milder than A and directionally better than B at the 95% confidence level.

EXAMPLE III

In a similar test as described in Example II, products with and without betaine and without betaine and citrate were tested. The results again show that the betaine and citrate are both necessary for optimum mildness.

| Composition % | A | B | C |
|---|---|---|---|
| $RE_xS$ | 27 | 27 | 27 |
| Dodecyldimethylamine oxide | 5 | 5 | 5 |
| Cetyldimethylbetaine | 0 | 0 | 5 |
| Sodium citrate | 0 | 6 | 6 |
| Relative Skin Grade Change* | Control | -0.25 | +0.34*** |
| Relative Dry Skin Grade Change** | Control | +0.25 | +0.67**** |

* A is used as the control. Higher numbers indicate better skin conditions.

** Dry Skin Grade

Dry skin grades are based upon a standard scale in which 6 is the worst grade and 0 is a perfect grade, measuring the amount of dry skin flakes on the skin surface.

*** Significantly better than A and B at the 90% confidence level.

**** Significantly better at the 90% confidence level versus the control and directionally better than B.

COMPARATIVE EXAMPLE IV

In a similar test as described in Example II, two other chelating agents were tested at equal chelating capacity to 6% sodium citrate.

| Composition % | A | B | C |
|---|---|---|---|
| $RE_x$-S | 27 | 27 | 27 |
| Dodecyldimethylamine oxide | 5 | 5 | 5 |
| Cetyldimethylbetaine | 0 | 5 | 5 |
| Disodium ethylenediamine-tetraacetate | 0 | 7.9 | 0 |
| Sodium polyacrylate (M.W. 10,000) | 0 | 0 | 3.2 |
| Relative Skin Grade Change | Control | -0.15 | -0.82 |
| Relative Dry Skin Grade Change | Control | +0.07 | -0.27 |

These chelating agents were not better and the polyacrylate was significantly worse than the control.

## EXAMPLE V

| Composition % | A | B | C | D | E | F | G | H | I | J |
|---|---|---|---|---|---|---|---|---|---|---|
| Sodium $C_{12-16}$ olefin sulfonate | 20 | 0 | 0 | 10 | 0 | 0 | 4 | 0 | 0 | 0 |
| Sodium $C_{10-16}$ alkyl-glycerylether sulfonate | 4 | 0 | 0 | 2 | 2 | 0 | 4 | 2 | 0 | 0 |
| Sodium $C_{12-16}$ olefin sulfate | 0 | 20 | 0 | 0 | 0 | 0 | 4 | 0 | 0 | 0 |
| Potassium $C_{12-16}$ alkyl-polyethoxylate$_{(6)}$ sulfate | 0 | 10 | 30 | 10 | 25 | 30 | 8 | 25 | 30 | 35 |
| $C_{12-16}$ alkylamidopropyl-dimethylbetaine | 0 | 4 | 6 | 0 | 4 | 2 | 6 | 0 | 6 | 0 |
| $C_{9-11}$ alkylbenzyldi-methylbetaine | 4 | 0 | 0 | 4 | 4 | 2 | 0 | 8 | 6 | 4 |
| $C_{10-16}$ alkyldi(2-hydroxy-ethyl)amine oxide | 4 | 6 | 2 | 0 | 0 | 4 | 2 | 0 | 0 | 6 |
| Potassium pyrophosphate | 2 | 0 | 0 | 0 | 0 | 0 | 0 | 0 | 4 | 0 |
| Ammonium maleate | 0 | 2 | 0 | 0 | 0 | 4 | 0 | 0 | 2 | 0 |
| Monoethanolammonium tartrate | 0 | 6 | 0 | 0 | 0 | 0 | 0 | 0 | 0 | 6 |
| Diethanolammonium succinate | 0 | 0 | 4 | 0 | 2 | 0 | 0 | 0 | 0 | 0 |
| Sodium malonate | 0 | 0 | 1 | 0 | 0 | 0 | 6 | 4 | 0 | 0 |

| | | | | | | | | | | |
|---|---|---|---|---|---|---|---|---|---|---|
| Sodium tripolyphosphate | 4 | 0 | 0 | 0 | 0 | 0 | 0 | 0 | 0 | 0 |
| Ammonium malate | 0 | 0 | 0 | 4 | 0 | 0 | 0 | 2 | 0 | 0 |
| Sodium saccharate | 0 | 0 | 0 | 0 | 5 | 2 | 0 | 0 | 0 | 0 |
| Citric acid | 2 | 2 | 2 | 2 | 0 | 2 | 2 | 0 | 0 | 0 |
| Water, ethanol, dyes, and materials to buffer pH at about 7. | Balance | | | | | | | | | |

All of the above compositions are mild to the skin.

## EXAMPLE VI

The test procedures of Examples II and III were used to evaluate compositions containing a base formula containing 13% sodium $C_{12-13}$ alkylethoxylate(1)sulfate; 14% sodium $C_{12-13}$ alkylpolyethoxylate(12) sulfate; and 4% $C_{12-14}$ fatty acid monoethanol amide and formulas containing the base formula plus the indicated additives.

| Formula | | Dry Skin Grade Change | Clinical Skin Grade Change |
|---|---|---|---|
| A | base + 5% cetyldimethyl betaine | 0.67 | -0.53 |
| B | base + 2% sodium citrate | 1.03 | -0.84 |
| C | base + 5% betaine + 2% citrate | 0.04 | -0.33 |
| Base | | 0.89 | -0.50 |
| | $LSD_{90}$ | 0.18 | 0.29 |

Both the dry skin grade and the clinical grade indicate that C is milder than the other compositions. High grades for the Dry Skin Grade indicate that the skin has become dryer; lower grades for the Clinical Skin Grades indicate the skin condition has changed less. The most important is the dry skin grade since it tends to reflect the consumer's perception better.

CLAIMS

1. A mild foaming detergent composition comprising:

(1) from about 5% to about 99% of mild, water soluble, foaming anionic detergent surfactant selected from the group consisting of: compounds having the formula $RE_xS$ wherein R is an alkyl group containing from about 10 to about 18 carbon atoms or an alkyl phenyl group in which the alkyl contains from about 5 to about 13 carbon atoms, E represents an ethylene oxide moiety, x is a number from about 1 to about 20 on the average, and S is a neutralized sulfate group; $C_{10-16}$ alkylglycerylether sulfonates; and mixtures thereof;

(2) from about 1% to about 30% of a betaine surfactant having the formula

$$R\text{-}\overset{(+)}{N}(R^1)_2\text{-}R^2CO\overset{(-)}{O}$$

wherein R contains from about 12 to about 20 carbon atoms, $R^1$ contains from one to about 3 carbon atoms and $R^2$ contains from one to about 3 carbon atoms;

(3) from 0% to about 20% of an amine oxide suds booster;

(4) from 0% to about 20% of a fatty acid amide suds booster; and

(5) from about 0.5% to about 50% of chelating agent which is effective at neutral or near neutral pH;

and, when said composition contains harsh surfactants such as $C_{10-16}$ alkyl sulfates and $C_{6-13}$ alkyl benzene sulfonates, said harsh surfactants are complexed with the amine oxide compound or the betaine surfactant to make them milder.

2. The composition of Claim 1 wherein in the betaine detergent surfactant the hydrophobic R contains from about 12 to about 18 carbon atoms and is selected from the group consisting of alkyl groups and alkyl groups interrupted by amido or ether linkages

and mixtures thereof; each $R^1$ is selected from the group consisting of methylethyl and hydroxyethyl groups; and $R^2$ is an alkylene group containing one carbon atom.

3. The composition of Claim 2 wherein R is an alkyl group containing from 12 to 18 carbon atoms.

4. The composition of Claim 3 wherein the betaine detergent surfactant is present at a level of from about 1% to about 15% and the ratio of anionic detergent surfactants to the betaine is from about 1:1 to about 30:1.

5. The composition of Claim 4 wherein the betaine is present at a level of from about 1% to about 10% and the ratio of anionic detergent surfactants to the betaine is from about 5:1 to about 20:1.

6. The composition of Claim 1 wherein the mild anionic detergent surfactant has the generic formula $RE_xSM$ wherein R is an alkyl group containing from about 12 to about 15 carbon atoms; E represents an ethylene oxide moiety; X is a number from about 1 to about 12 on the average; S is a sulfate group and M is selected from the group consisting of alkaline metal, alkaline earth metal, ammonium and substituted ammonium cation; wherein the amine oxide suds booster has the formula

$$R^1-(OR^2)_n-N(R^3)_2 \rightarrow O$$

in which $R^1$ is an alkyl group of from about 8 to about 18 carbon atoms; $R^2$ is an alkylene or a hydroxy alkylene group containing 2 to 3 carbon atoms; n ranges from 0 to about 20; and each $R^3$ is selected from the group consisting of methyl, ethyl and hydroxyethyl groups which can be joined to form morpholine or pyridine rings, and mixtures thereof; and wherein the amount of anionic detergent is from about 10% to about 40% and the amount of the amine oxide is from about 1% to about 15%.

7. The composition of Claim 6 wherein R is an alkyl group containing from 12 to 18 carbon atoms.

8. The composition of Claim 7 wherein the betaine detergent surfactant is present at a level of from about 1% to about 15% and the ratio of anionic detergent surfactants to the betaine is from about 1:1 to about 20:1.

9. The composition of Claim 8 wherein the betaine is present at a level of from about 1% to about 10% and the ratio of anionic detergent surfactants to the betaine is from about 3:1 to about 10:1.

10. The composition of Claim 1 containing from about 20% to about 35% of the mild anionic detergent surfactant and from about 1% to about 20% of the chelating agent.

11. The composition of Claim 10 wherein the amount of the chelating agent is from about 1% to about 10%.

12. The composition of Claim 11 wherein the ratio of mild anionic detergent surfactant to amine oxide is from about 3:1 to about 10:1.

13. The composition of Claim 1 wherein the chelating agent is a water soluble citric acid or salt.

14. The composition of Claim 13 wherein in the betaine detergent surfactant the hydrophobic R contains from about 12 to about 18 carbon atoms and is selected from the group consisting of alkyl groups and alkyl groups interrupted by amido or ether linkages and mixtures thereof; each $R^1$ is selected from the group consisting of methyl, ethyl and hydroxyethyl groups; and $R^2$ is an alkylene group containing one carbon atom.

15. The composition of Claim 14 wherein the betaine detergent surfactant is present at a level of from about 1% to about 15% and the ratio of anionic detergent surfactants to the betaine is from about 1:1 to about 20:1.

16. The composition of Claim 15 wherein the betaine is present at a level of from about 1% to about 10% and the ratio of anionic detergent surfactants to the betaine is from about 3:1 to about 10:1.

17. The composition of Claim 1 wherein the chelating agent is selected from the group consisting of the salts of citric acid, malic acid, saccharic acid, maleic acid, succinic acid, malonic acid, tartaric acid, ether polycarboxylates and mixtures thereof.

18. The composition of Claim 17 wherein the chelating agent comprises an ether polycarboxylate having the general formula:

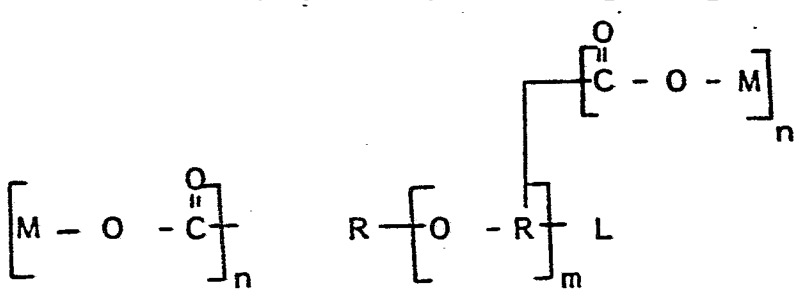

wherein each R is an alkylene or hydroxy alkylene group containing from 0 to about 3 hydroxy groups, each n is from one to about 4, m is from one to about 4, each M is H or a water soluble cation, and L is -H, -OH, or a covalent bond linking the last R group to an earlier R group to complete the formula.

19. The composition of Claim 1 containing from about 1% to about 15% fatty acid amide suds booster having the formula:

$$R-\overset{\overset{O}{\|}}{C}-N(H)_x(R^1)_{2-x}$$

wherein R is an alkyl chain containing from about 7 to about 17

carbon atoms; X is 0, 1 or 2; and each $R^1$ is an alkyl or hydroxy alkyl group containing from 1 to 3 carbon atoms, the ratio of anionic surfactant to amide being from about 2:1 to about 20:1.

20. The composition of Claim 20 wherein the acyl group in the fatty acid amide suds booster contains from about 12 to about 14 carbon atoms and at least one $R^1$ is either an hydroxyethyl or hydroxypropyl group and any remaining $R^1$ is H.

RBA:sp(3364R/A19)